# EUROPÄISCHE PATENTANMELDUNG

(11) **EP 2 364 737 A1**
(43) Veröffentlichungstag der Anmeldung: **14.09.2011**
(21) Anmeldenummer: 10155838.5
(22) Anmeldetag: 08.03.2010
(51) Int. Cl.: A61M 5/168, G01L 3/14

(54) **Antriebssystem**

(71) Anmelder: Fresenius Kabi Deutschland GmbH, 61352 Bad Homburg (DE)
(72) Erfinder: Krater, Jörg, 99974 Reiser (DE)
(74) Vertreter: Richly, Erik

(57) **Zusammenfassung**

Die Erfindung betrifft ein Antriebssystem mit einem Antrieb, einer Kupplung und einer Last, wobei die Last über die Kupplung mit dem Antrieb verbunden ist.

Das erfindungsgemäße Antriebssystem umfasst einen Antrieb (1), eine Last (2), eine Kupplung (3) und eine Messvorrichtung (4), wobei die Kupplung (3) derart eingerichtet ist, dass diese eine von dem Antrieb (1) erzeugte Bewegung auf die Last (2) überträgt, wobei die Kupplung (3) ein antriebsseitiges erstes Kupplungselement (5) und ein lastseitiges zweites Kupplungselement (6) aufweist, und die Kupplung (3) derart eingerichtet ist, dass das erste Kupplungselement (5) und das zweite Kupplungselement (6) ihre Stellung in Abhängigkeit einer von der Last (2) auf die Kupplung (3) ausgeübten Belastung zueinander verändern, und die Messvorrichtung (4) derart eingerichtet ist, dass diese eine Änderung der Stellung des ersten Kupplungselementes (5) relativ zum zweiten Kupplungselement (6) misst.

## Beschreibung

### Technisches Gebiet

Die Erfindung betrifft ein Antriebssystem mit einem Antrieb, einer Kupplung und einer Last, wobei die Last über die Kupplung mit dem Antrieb verbunden ist. Insbesondere betrifft die Erfindung einen Antrieb für medizinische Pumpen.

### Stand der Technik

Antriebssysteme, die einen Antrieb, eine Kupplung und eine über die Kupplung mit dem Antrieb verbundene Last aufweisen sind allgemein bekannt. Beispielsweise sind in der Medizintechnik Antriebssysteme bekannt, die einen Elektromotor, eine Kupplung und eine von dem Elektromotor über die Kupplung angetriebene Pumpe aufweisen. Im Bereich der enteralen und der parenteralen Ernährung werden beispielsweise Peristaltikpumpen oder Rollenpumpen eingesetzt, die eine kontinuierliche Förderung einer Ernährungslösung ermöglichen. Die Förderung der Ernährungslösung erfolgt in diesem Fall in Zusammenwirkung der Pumpe mit einer Zuführleitung, üblicherweise einem Schlauch.

Um einen Fehler, beispielsweise eine Okklusion, in einer für die Zuführung der Ernährungslösung oder auch von Medikamenten verwendeten Zuführleitung zu detektieren, ist es bekannt, Drucksensoren einzusetzen, die den Druck innerhalb der Zuführleitung messen. Eine Okklusion lässt sich dadurch erkennen, dass der gemessene Druck außerhalb eines üblichen Wertebereiches liegt. Als Drucksensoren, die innerhalb der Zuführleitung den Druck messen können, sind beispielsweise Piezoelemente, Dehnungsmessstreifen oder kapazitive Sensoren bekannt. Messvorrichtungen, die es ermöglichen, zuverlässig den Druck innerhalb der Zuführleitung zu messen, sind allerdings mit deutlichen Kosten verbunden. Aufgrund dessen wird beispielsweise bei enteralen Pumpen, bei denen eine derartige Überwachung nicht zwingend vorhanden sein muss, auf derartige Messvorrichtungen verzichtet.

Die US2002042953A1 offenbart ein Antriebssystem mit einem Gleichstromelektromotor und einer Spritzenpumpe. Das Antriebsystem ist derart eingerichtet, dass der Motorstrom der Gleichstromelektromotors gemessen wird, während der Gleichstrommotor die Spritzenpumpe antreibt. Der gemessene Motorstrom gibt die durch die Spritzenpumpe auf den Gleichstromelektromotor wirkende Belastung wieder. Überschreitet der gemessene Motorstrom einen Schwellenwert, wird auf eine Okklusion erkannt und ein Alarm ausgelöst.

### Kurzbeschreibung der Erfindung

Das erfindungsgemäße Antriebssystem umfasst einen Antrieb, eine Last, eine Kupplung und eine Messvorrichtung, wobei die Kupplung derart eingerichtet ist, dass diese eine von dem Antrieb erzeugte Bewegung auf die Last überträgt, wobei die Kupplung ein antriebsseitiges erstes Kupplungselement und ein lastseitiges zweites Kupplungselement aufweist, und die Kupplung derart eingerichtet ist, dass das erste Kupplungselement und das zweite Kupplungselement ihre Stellung in Abhängigkeit einer von der Last auf die Kupplung ausgeübten Belastung zueinander verändern, und die Messvorrichtung derart eingerichtet ist, dass diese eine Änderung der Stellung des ersten Kupplungselementes relativ zum zweiten Kupplungselement misst.

Die Kupplung in Verbindung mit der Messvorrichtung ermöglicht eine kostengünstige und zuverlässige Messung der an der Kupplung anliegenden Belastung. Ist beispielsweise die Last eine Pumpe, können mittels der erfindungsgemäßen Kupplung und Messvorrichtung unterschiedliche Belastungszustände der Pumpe erfasst werden. Im Falle einer Rollenpumpe oder Peristaltikpumpe kann beispielsweise eine sich in einer mit der Pumpe in Verbindung stehenden Zuführleitung auftretende Druckänderung erkannt werden. Beispielsweise ist es auf diese Weise möglich, eine Okklusion in der Zuführleitung zu erkennen. Des Weiteren ist dadurch, dass die Messung der Belastung innerhalb der Messkette nach dem Antrieb erfolgt, der Antrieb aus der Messkette eliminiert. Damit können Änderungen der Eigenschaften des Antriebs, die beispielsweise durch Abnutzungserscheinungen eines zum Antrieb gehörenden Motors und/oder einer Getriebeeinheit verursacht werden können, das Messergebnis nicht beeinflussen und somit als Fehlerquelle ausgeschlossen werden.

Vorzugsweise ist das Antriebssystem derart eingerichtet, dass das erste Kupplungselement und das zweite Kupplungselement bei einer von der Last auf die Kupplung ausgeübten Belastung von einer Ausgangsstellung in eine Belastungsstellung übergehen, in der die Stellung des ersten Kupplungselementes relativ zum zweiten Kupplungselement verändert ist, und bei einem Wegfall der Belastung das erste Kupplungselement und das zweite Kupplungselement in ihre Ausgangsstellung zurückkehren. Dadurch, dass die Kupplungselemente von einer Ausgangsstellung in eine Belastungsstellung und zurück in die Ausgangsstellung kehren können, kann gewährleistet werden, dass das erste Kupplungselement und das zweite Kupplungselement abhängig von der Belastung definierte relative Stellungen einnehmen. Beispielsweise nehmen bei einer erneuten Belastung, die der vorherigen Belastung entspricht, das erste Kupplungselement und das zweite Kupplungselement die vorhergehende relative Stellung ein. Damit können der relativen Stellung von ersten Kupplungselement und zweiten Kupplungselement Werte zugeordnet werden, die die durch die Last auf die Kupplung ausgeübte Belastung eindeutig wiedergeben.

Eine weitere vorteilhafte Weiterbildung der Erfindung sieht vor, dass das erste Kupplungselement und das zweite Kupplungselement durch mindestens ein Federelement miteinander und gegeneinander beweglich verbunden sind, wobei das mindestens eine Federelement bei einer von der Last auf die Kupplung ausgeübten Belastung elastisch deformiert wird. Dadurch, dass erstes Kupplungselement und zweites Kupplungselement miteinander über mindestens ein elastisch deformierbares Federelement verbunden sind, kann gewährleistet werden, dass das erste Kupplungselement und das zweite Kupplungselement in Abhängigkeit der auf die Kupplung wirkenden Belastung zueinander definierte, eindeutige Stellungen einnehmen. Insbesondere kann durch das Federelement eine Rückstellung des ersten und des zweiten Kupplungselementes bewirkt werden. Der Begriff "Federlement" soll in diesem Zusammenhang alle elastisch deformierbaren Elemente umfassen, beispielsweise Druckfedern, Zugfedern, Torsionsfedern, Elastomerfedern, Biegefedern, Luftfedern oder Gasdruckfedern, wobei diese Aufzählung nicht abschließend ist.

Eine weitere vorteilhafte Weiterbildung sieht vor, dass das Antriebssystem zusätzlich eine Steuervorrichtung umfasst, wobei die Steuereinrichtung derart eingerichtet ist, dass diese überprüft, ob ein von der Messvorrichtung gemessener Wert in einem vorgegebenen Wertebereich liegt, und im Falle dessen, dass der Wert außerhalb des Wertebereiches liegt, die vom Antrieb erzeugte Bewegung reduziert wird, die vom Antrieb erzeugte Bewegung stoppt und/oder ein Signal abgibt. Über die Auswertung der von der Messvorrichtung gemessenen Messwerte lassen sich insbesondere lastseitige Anormalitäten erkennen. Beispielsweise kann auf diese Weise eine Okklusion in einer Zuführleitung erkannt werden, die sich durch eine erhöhte Belastung der mit der Zuführleitung verbundenen Pumpe und damit einer auf die Kupplung erhöhten Belastung äußert. Beispielsweise kann der Wertebereich durch einen in der Steuervorrichtung hinterlegten Schwellenwert definiert sein, der einem einer Okklusion entsprechenden Belastungswert entspricht. Beim Überschreiten des Schwellenwertes können durch die Steuervorrichtung verschiedene Maßnahmen eingeleitet werden, beispielsweise ein Abschalten oder Reduzierung des Antriebs und/oder eine Signalisierung, beispielsweise akustisch oder visuell.

Die abhängigen Ansprüche geben weitere vorteilhafte Weiterbildungen der Erfindung an.

Im Folgenden wird die Erfindung anhand von Ausführungsbeispielen, die durch mehrere Figuren dargestellt sind, näher beschrieben.

### Kurze Beschreibung der Zeichnungsfiguren

Es zeigt:

Fig. 1 eine schematische Darstellung einer Ausführungsform eines erfindungsgemäßen Antriebssystems,

Fig. 2 ein von der Messvorrichtung des Antriebssystems aufgenommenes Signal in Abhängigkeit der Zeit bei einer Belastung der Kupplung des Antriebssystems in einer ersten Belastungshöhe,

Fig. 3 ein von der Messvorrichtung des Antriebssystems aufgenommenes Signal in Abhängigkeit der Zeit bei einer Belastung der Kupplung in einer sich von der ersten unterscheidenden zweiten Belastungshöhe,

Fig. 4 eine perspektivische Ansicht eines Ausschnitts des Antriebssystems,

Fig. 5 eine weitere perspektivische Ansicht des Antriebssystems,

Fig. 6 eine perspektivische Ansicht des ersten und zweiten Kupplungselements des Antriebssystems,

Fig. 7 eine perspektivische Ansicht des ersten Kupplungselementes,

Fig. 8 eine alternative Ausführungsform der Kupplung des Antriebssystems in einer Draufsicht,

Fig. 9 die in Fig. 8 gezeigte alternative Ausführungsform der Kupplung in einer Seitenansicht,

Fig. 10 das erste Kupplungselement der in Fig. 8 gezeigten Kupplung in einer perspektivischen Ansicht,

Fig. 11 das zweite Kupplungselement der in Fig. 8 gezeigten Kupplung in einer perspektivischen Ansicht,

Fig. 12 eine weitere alternative Ausführungsform einer Kupplung des erfindungsgemäßen Antriebssystems in einer Draufsicht,

Fig. 13 die in Fig. 12 gezeigte alternative Ausführungsform der Kupplung in einer perspektivischen Ansicht,

Fig. 14 die in Fig. 12 gezeigte alternative Ausführungsform der Kupplung in weiteren perspektivischen Ansicht, und

Fig. 15 eine weitere Ausführungsform einer Kupplung des erfindungsgemäßen Antriebssystems in einer schematischen Darstellung.

**Beschreibung der Ausführungsformen**

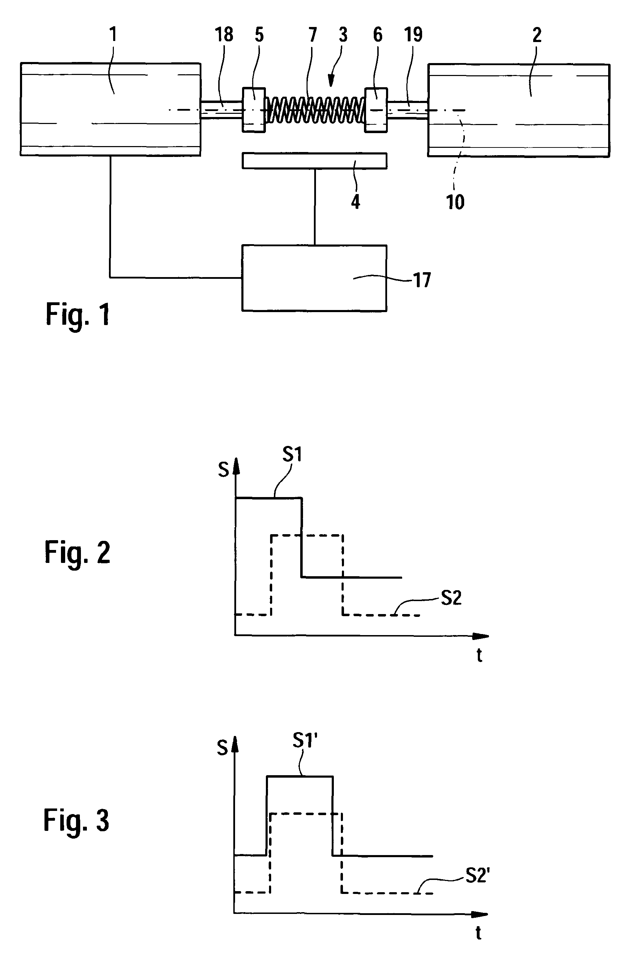

Die Fig. 1 zeigt schematisch eine Ausführungsform eines erfindungsgemäßen Antriebssystems. Das Antriebssystem umfasst einen Antrieb 1, eine Last 2, eine Kupplung 3, eine Messvorrichtung 4 und eine Steuervorrichtung 17. Die Kupplung 3 ist derart eingerichtet, dass diese eine von dem Antrieb 1 erzeugte Bewegung auf die Last 2 überträgt. Die Kupplung 3 umfasst ein antriebsseitiges erstes Kupplungselement 5 und ein lastseitiges Kupplungselement 6. Das erste Kupplungselement 5 und das zweite Kupplungselement 6 sind in diesem Ausführungsbeispiel durch mindestens ein elastisch deformierbares Federelement verbunden, wobei das Federelement in der Fig. 1 symbolisch durch eine Schraubenfeder 7 dargestellt ist. Die Messvorrichtung 4 ist derart eingerichtet, dass diese eine Änderung der Stellung des ersten Kupplungselementes 5 relativ zum zweiten Kupplungselement 6 misst. Die von der Messvorrichtung 4 aufgenommenen Messwerte werden an die Steuereinheit 17 übertragen und von dieser ausgewertet. Abhängig von dem Ergebnis der Auswertung kann die Steuereinheit 17 Maßnahmen einleiten, beispielsweise ein Signal abgeben und/oder den Antrieb 1 stoppen und/oder den Antrieb 1 in seiner Antriebsgeschwindigkeit reduzieren. Andere Maßnahmen sind ebenfalls denkbar.

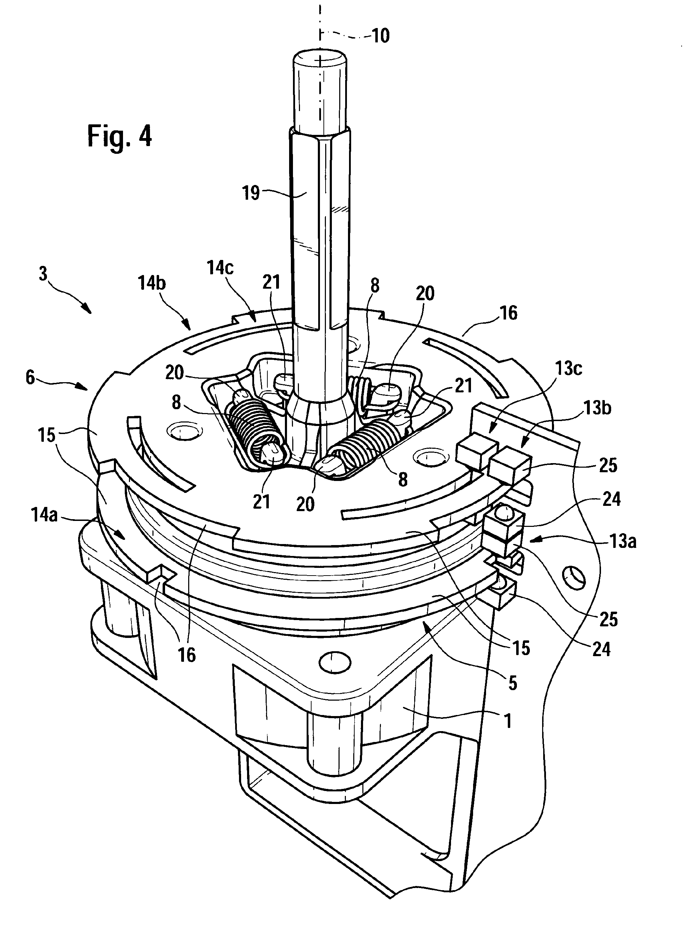

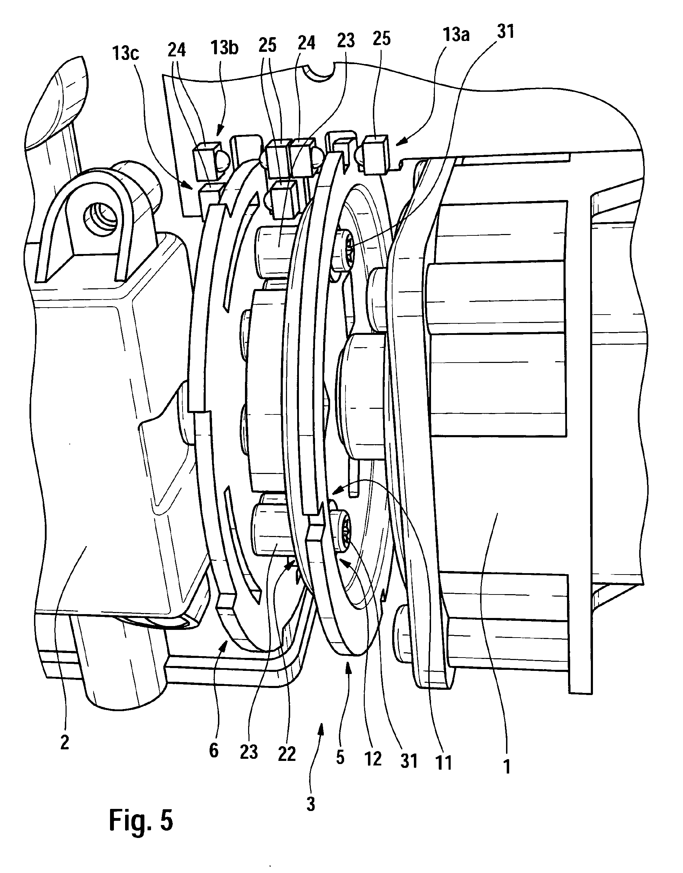

Die Figuren 4 und 5 zeigen einen Ausschnitt des Antriebsystems in zwei perspektivischen Ansichten.

In diesem Ausführungsbeispiel sind das erste Kupplungselement 5 und das zweite Kupplungselement 6 scheibenförmig mit im Wesentlichen übereinstimmenden Außendurchmessern ausgebildet. Ein scheibenförmiger Aufbau zumindest eines der Kupplungselemente 5, 6 ermöglicht einen platzsparenden und kompakten Aufbau der Kupplung 3. Das erste Kupplungselement 5 ist in seinem Zentrum fest mit einer ersten Welle 18 (in Fig. 1 schematisch dargestellt) des Antriebs 1 verbunden, das zweite Kupplungselement 6 ist fest mit einer zur Last 2 gehörenden zweiten Welle 19 verbunden.

Das erste Kupplungselement 5 und das zweite Kupplungselement 6 sind gegenüberliegend angeordnet. Die Kupplung 3 umfasst drei Federelemente, hier drei Zugfedern 8, die in einer im Wesentlichen gemeinsamen Ebene in einem ausgesparten Innenbereich des zweiten Kupplungselementes 6 umlaufend um eine Drehachse 10 angeordnet und mit einem Ende mit dem ersten Kupplungselement 5 und mit dem anderen Ende mit dem Kupplungselement 6 verbunden sind. Dies ermöglicht es, das erste Kupplungselement 5 gegen das zweite Kupplungselement 6 aus einer Ausgangsstellung gegen eine zunehmende Rückstellkraft der Zugfedern 8 in einem Winkelbereich kontinuierlich zu verdrehen. Das eine Ende einer Zugfeder 8 ist jeweils mit einem ersten Zapfen 21 des ersten Kupplungselementes 5, das andere Ende einer Zugfeder 8 mit einem zweiten Zapfen 20 des zweiten Kupplungselementes 6 einhakend verbunden.

Alternativ könnte nur eine oder zwei Zugfedern 8 eingesetzt werden, ebenso ist es möglich, mehr als drei Zugfedern 8 zu verwenden. Die Zugfedern 8 können in der Ausgangsstellung entspannt sein, oder, was bevorzugt ist und wie es in diesem Ausführungsbeispiel umgesetzt ist, vorgespannt sein. Die Vorspannung bestimmt die mittels der Kupplung 3 minimal messbare Kraft bzw. das minimal messbare Drehmoment.

Die Zugfedern 8 bewirken eine Kraftübertragung zwischen dem ersten Kupplungselement 5 und dem zweiten Kupplungselement 6. Des Weiteren ermöglicht diese Art der Kraftübertragung einen Versatz der Drehachsen des ersten Kupplungselementes 5 und des zweiten Kupplungselementes 6, wodurch durch die Kupplung 3 beispielsweise Fertigungstoleranzen und/oder Montagetoleranzen zwischen Antrieb 1 und Last 2 ausgeglichen werden können. Die Fig. 4 stellt den idealen Fall dar, das erste Kupplungselement 5 und zweites Kupplungselement 6 auf einer gemeinsamen Drehachse 10 liegen. Im Rahmen dieser Erfindung soll allerdings auch ein leichter Versatz der den Kupplungselementen 5, 6 zugeordneten Drehachsen um höchstens 10°, vorzugsweise um höchstens 5° als "auf einer gemeinsamen Drehachse angeordnet" aufgefasst werden.

Eine durch den Antrieb 1 erzeugte Drehbewegung wird über die Kupplung 3 auf die Last 2 übertragen. Das Umdrehungsverhältnis der Kupplung 3 ist, bedingt durch den Aufbau der Kupplung 3, motorseitig und lastseitig 1:1. Eine lastseitig ausgeübte Belastung der Kupplung 3 bewirkt gegen die Rückstellkraft der Zugfedern 8 eine Verdrehung der beiden Kupplungselemente 5, 6 aus der Ausgangsstellung um einen belastungsabhängigen Winkel in eine Belastungsstellung. Die Zugfedern 8 bewirken bei einer Reduzierung der Belastung die Rückstellung der beiden Kupplungselemente 5, 6, bei Wegfall der Belastung insbesondere die Rückstellung in die Ausgangsstellung. Innerhalb des Winkelbereiches, in dem sich das erste Kupplungselement 5 und das zweite Kupplungselement 6 gegeneinander verdrehen können, kann auf diese Weise jeder Stellung der Kupplungselemente 5, 6 ein Belastungswert eindeutig zugeordnet werden. Im Falle von vorgespannten Zugfedern 8 erfolgt die Änderung der relativen Stellung der Kupplungselemente 5, 6 erst, nachdem die auf die Kupplung 3 ausgeübte Belastung die Rückstellkraft der Zugfedern 8 überschritten hat.

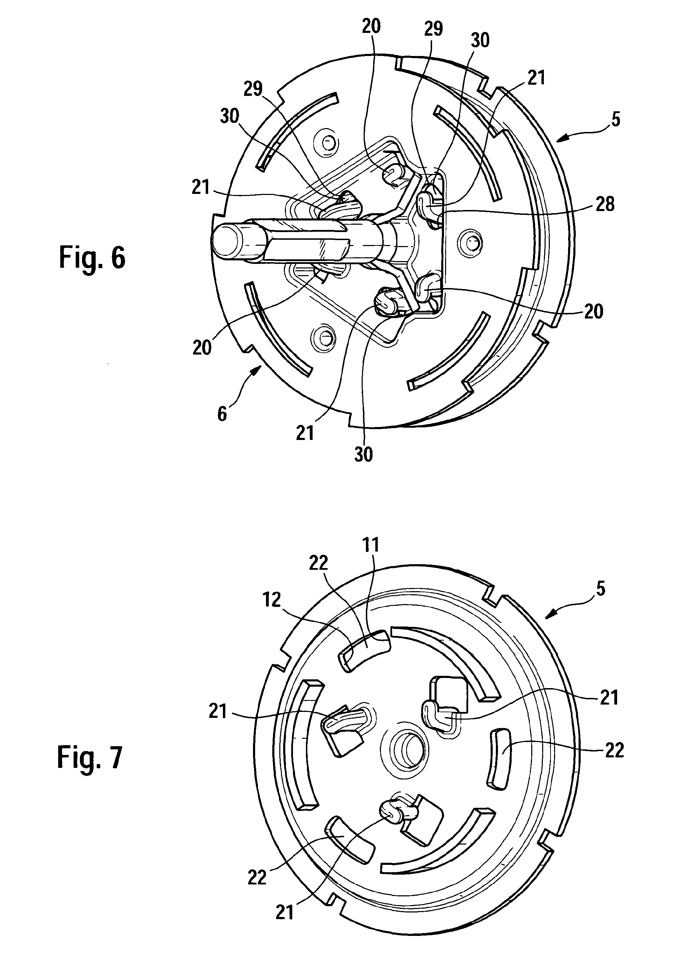

Die Kupplung 3 umfasst einen Anschlag, der die relative Verdrehung des ersten Kupplungselemente 5 und des zweiten Kupplungselemente 6 begrenzt. Der Anschlag wird durch die jeweiligen beiden Enden 28, 29 dreier länglicher Öffnungen 30 im zweiten Kupplungselement 6 gebildet, siehe Figuren 6, 7. Eine Öffnung 30 wird jeweils durch einen ersten Zapfen 21 durchgriffen. Im unbelasteten Zustand der Kupplung 3 werden die ersten Zapfen 21 durch die Rückstellkraft der Zugfedern 8 gegen das jeweilige erste Ende 28 gedrückt, beim Verdrehen der Kupplungselemente 5, 6 werden die ersten Zapfen 21 gegen die Rückstellkraft der Zugfedern 8 gegen das zweite Ende 29 bewegt. Der Anschlag verhindert eine Überbeanspruchung oder Unterbelastung der Zugfedern 8.

Das erste Kupplungselement 5 umfasst drei Kulissen 22. Die Kulissen 22 umlaufen die vorgesehene Drehachse 10 abschnittsweise auf einer Kreisbahn. Das zweite Kupplungselement 6 weist drei Schraubdome 23 mit in den Schraubdomen 23 eingeschraubten Zylinderkopfschrauben 31 auf, wobei jeweils eine Schraube 31 eine Kulisse 22 durchgreift. Die Schrauben 31 und die Schraubdome 23 über- bzw. untergreifen mit Spiel die Kulissen 22, so dass das erste Kupplungselement 5 und das zweite Kupplungselement 6 sich in axialer Richtung nicht lösen können, aber eine geringe Abweichung der Drehachsen der Kupplungselemente 5, 6 ausgeglichen werden kann. Die Schrauben 23 in Verbindung mit den Kulissen 22 dienen als Montagehilfe, im Betrieb der Kupplung 3 wird ein Kontakt zwischen Schrauben 23 und Kulisse 22 weitestgehend, bevorzugt vollständig, vermieden. Es ist bevorzugt, dass ein Kontakt der beiden Kupplungselemente 5, 6 lediglich durch das Federelement, hier durch die Zugfedern 8, hergestellt ist, um Reibungsverluste zwischen den Kupplungselementen 5, 6 zu minimieren.

Alternativ oder zusätzlich können die Kulissen 22 in Verbindung mit den Schrauben 23 als Anschläge ausgebildet sein, die die Verdrehung des ersten Kupplungselementes 5 gegen das zweite Kupplungselement 6 in eine oder in beide Richtungen begrenzen. Anstatt durch Schraubdome 23 und Schrauben 31 können diese Funktionen selbstverständlich auch durch andere Elemente verwirklicht werden, beispielsweise durch Zapfen, die entsprechend den Schraubdomen 23 an dem zweiten Kupplungselement angeordnet sind und die Kulissen 22 durchgreifen.

Die Messvorrichtung 4 umfasst drei Lichtschranken 13a, 13b, 13c, mittels derer die relative Stellung des ersten Kupplungselementes 4 und des zweiten Kupplungselemente 6 bestimmbar sind. Die Lichtschranken 13a, 13b, 13c umfassen jeweils eine Lichtquelle 24 und eine der jeweiligen Lichtquelle gegenüberliegenden Photodiode 25. Die erste Lichtschranke 13a ist an einer ersten Spur 14a des ersten Kupplungselementes 5 angeordnet, die zweite Lichtschranke 13b an einer zweiten Spur 14b und die dritte Lichtschranke 13c an einer dritten Spur 14c des zweiten Kupplungselementes 6 angeordnet. Die Spuren 14a, 14b, 14c sind im Randbereich der Kupplungselemente 5, 6 angeordnet und werden jeweils durch auf einer Kreisbahn verlaufende, sich abwechselnde geschlossene Bereiche 15 und offene Bereiche 16 gebildet. Bei einer Drehung der Kupplungselemente 5, 6 werden die Lichtschranken 13a, 13b, 13c in durch die Ausbildung der offenen Bereiche 15 und geschlossenen Bereiche 16 bestimmten Zeitintervallen abwechselnd geöffnet und geschlossen, wobei die mit dem Öffnen und Schließen verbundene zeitabhängige Signaländerung durch die Messvorrichtung 4 detektiert wird.

Die Fig. 2 zeigt ein erstes Diagramm, in dem das mittels der ersten Lichtschranke 13a gemessene Signal S1 und das mittels der zweiten Lichtschranke 13b gemessene Signal S2 bei einer durch den Antrieb 1 bewirkten konstanten Drehbewegung der Kupplungselemente 5, 6 über die Zeit aufgetragen sind. Der Übersicht wegen sind die Signale S1, S2 in ihrem Signalwert versetzt dargestellt. Ist die jeweilige Lichtschranke 13a, 13b offen, so erhöht sich der gemessene Signalwert der Signale S1, S2, wird die Lichtschranke 13a, 13b geschlossen, so erniedrigt sich der Signalwert der Signale S1, S2. Der Signalverlauf ist abhängig von der Ausbildung der Spuren 14a, 14b und der Winkelgeschwindigkeit der Kupplungselemente 5, 6.

Ändert sich die auf die Kupplung 3 wirkende Belastung und bewirkt damit eine Verdrehung des ersten Kupplungselements 5 relativ zum zweiten Kupplungselement 6, so äußert sich dies in einer zeitlichen Verschiebung bzw. Phasenverschiebung der Signale S1, S2 zu Signalen S1', S2', siehe Figur 3. Die relative zeitliche Verschiebung bzw. Phasendifferenz ist abhängig von der Höhe der auf die Kupplung 3 wirkenden Belastung. Die relative zeitliche Verschiebung der Signale S1, S2 kann auf diese Weise eindeutig einer von der Last 2 auf die Kupplung 3 ausgeübten Belastung zugeordnet werden.

Alternativ kann eine Lichtschranke verwendet werden, die sowohl durch die erste Spur 14a des ersten Kupplungselements 5 als auch durch die zweite Spur 14b des zweiten Kupplungselementes 6 geöffnet und geschlossen werden kann. In diesem Falle sind die offenen und geschlossenen Bereiche 15, 16 der Spuren 14a, 14b derart ausgebildet, dass eine Verdrehung des ersten Kupplungselementes 5 relativ zum zweiten Kupplungselement 6 eine Änderung der geschlossenen und offenen Bereiche der sich überlappenden Spuren 14a, 14b bewirkt. Die sich bei einer Drehung der Kupplung 3 in Abhängigkeit der Belastung ändernden Zeitintervalle, in denen die Lichtschranke geöffnet oder geschlossen ist, resultieren in einem belastungsabhängigen Signalverlauf, durch dessen Auswertung auf die durch die Last 2 auf die Kupplung 3 wirkende Belastung geschlossen werden kann.

Die dritte Spur 14c, die um einen Winkel versetzt zur zweiten Spur 14b angeordnet ist, ermöglicht in Verbindung mit der dritten Lichtschranke 13c eine zusätzliche Bestimmung der Stellung des zweiten Kupplungselementes 6. In Kombination mit der zweiten Lichtschranke 13b und der zweiten Spur 14b ist auf diese Weise eine verbesserte Bestimmung der absoluten Stellung des zweiten Kupplungselementes 6 möglich. Die Bestimmung der Stellung des zweiten Kupplungselementes 6 ermöglicht es, den Zustand der Last 2, beispielsweise die momentane Stellung einer als Last 2 eingesetzten Pumpe, zu bestimmen. Durch die Erhöhung der Anzahl der geschlossenen und offenen Bereiche auf einer Spur kann die Genauigkeit einer derartigen Positionsbestimmung ebenfalls erhöht werden.

Alternativ zu einer Lichtschranke 13a, 13b, 13c kann beispielsweise auch ein oder mehrere Hall-Sensoren eingesetzt werden. Die Spuren 14a, 14b, werden in diesem Falle durch sich abwechselnde magnetische und nichtmagnetische Bereiche gebildet. Des Weiteren ist alternativ oder zusätzlich die Verwendung von kapazitiven oder taktilen Sensoren möglich.

Die von der Messvorrichtung 4 aufgenommen Signale werden zwecks Auswertung an die Steuervorrichtung 17 weitergeleitet. Die Steuervorrichtung 17 ist in diesem Falle Teil des Antriebssystems, kann alternativ beispielsweise auch eine externe Vorrichtung sein. Messvorrichtung 4 und Steuervorrichtung 17 können getrennt oder zumindest teilweise in einer gemeinsamen Elektronik integriert sein. Die Steuervorrichtung 17 ist derart eingerichtet, dass diese überprüft, ob ein von der Messvorrichtung gemessener Wert in einem vorgegebenen Wertebereich liegt, und im Falle dessen, dass der Wert außerhalb des Wertebereiches liegt, eine oder mehrere Maßnahmen einleitet. Der Wertebereich kann durch einen minimalen Wert und/oder einen maximalen Wert, insbesondere einen unteren Schwellenwert und/oder einen oberen Schwellenwert definiert sein. Die Steuervorrichtung 17 umfasst für die Auswertung der Messdaten vorzugsweise eine CPU, die angegebenen Werte sind vorzugsweise in einem Speicher der Steuervorrichtung 17 hinterlegt.

Beispielsweise ist in der Steuervorrichtung 17 ein Schwellenwert hinterlegt, der eine maximale Belastung der Kupplung 3 durch die Last 2 wiedergibt. Es können auch mehrere Schwellenwerte hinterlegt sein, die mehrere Laststufen der Kupplung 3 durch die Last 2 wiedergeben. Die Steuervorrichtung 17 ist derart eingerichtet, dass diese im Betrieb des Antriebssystems kontinuierlich überprüft, ob die aus der Auswertung der von der Messeinrichtung 5 gemessenen Signale resultierenden Belastungswerte den Schwellenwert bzw. die Schwellenwerte überschreiten. Ist dies der Fall, werden durch die Steuervorrichtung 17 Maßnahmen eingeleitet, beispielsweise das Abschalten des Antriebs oder eine Reduktion der Antriebsgeschwindigkeit, und/oder die Abgabe eines Signals, beispielsweise eines akustischen Signals oder eines visuellen Signals. Alternativ oder zusätzlich kann der Messwert angezeigt werden, beispielsweise als zeitabhängiges Drehmoment.

Der Antrieb 1 ist hier ein elektrisch betriebener Schrittmotor. Ein Schrittmotor stellt einen besonders kostengünstigen Antrieb 1 dar. Alternativ ist beispielsweise auch ein Gleichstrommotor, sei es mit einer Kontaktbürste oder bürstenlos, einsetzbar. In letzterem Falle kann der Motorstrom verwendet werden, um eine durch die Last 2 verursachte Belastung zusätzlich zu messen. Grundsätzlich kann der Antrieb 1 durch eine beliebige Art von Motor gebildet werden.

Die Last 2 ist in diesem Ausführungsbeispiel eine medizinische Pumpe. Die Pumpe kann insbesondere eine Rollenpumpe oder eine Peristaltikpumpe sein. Grundsätzlich ist das beschriebene Antriebssystem mit jeder Art von Last 2 verwendbar.

Der in der Steuervorrichtung 17 hinterlegte Schwellenwert entspricht dem Wert einer von der medizinischen Pumpe auf die Kupplung 3 ausgeübten Belastung, falls in einer mit der medizinischen Pumpe verbundenen Zuführleitung eine Okklusion auftritt. Tritt eine Okklusion in der Zuführleitung auf, überschreitet der aus den Messsignalen erhaltene Belastungswert den Schwellenwert, wodurch die Steuervorrichtung 17 auf Okklusion erkennt. Anschließend können beispielsweise oben beschriebene Maßnahmen eingeleitet werden.

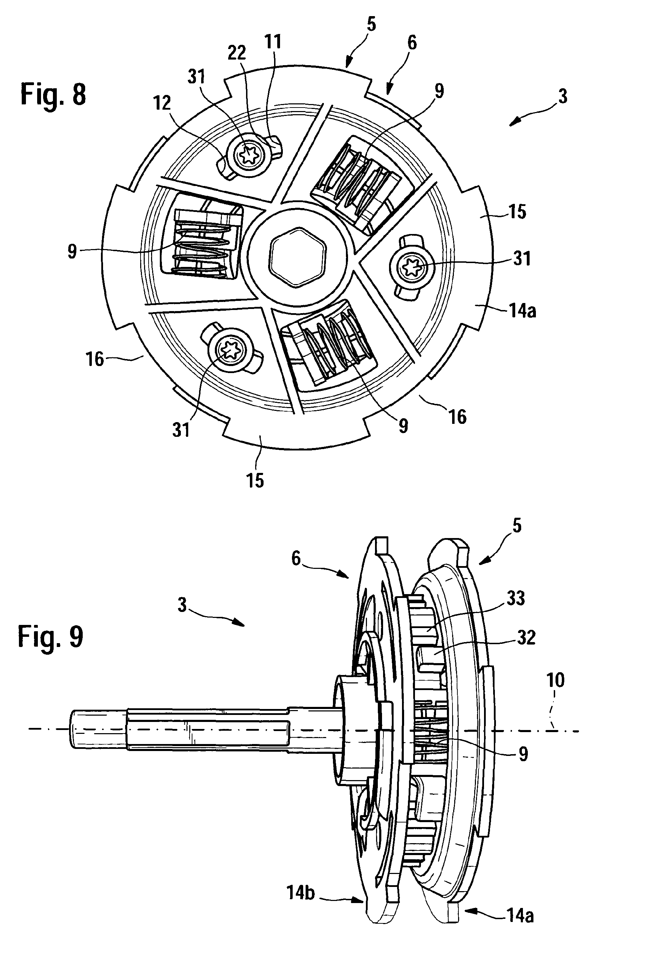

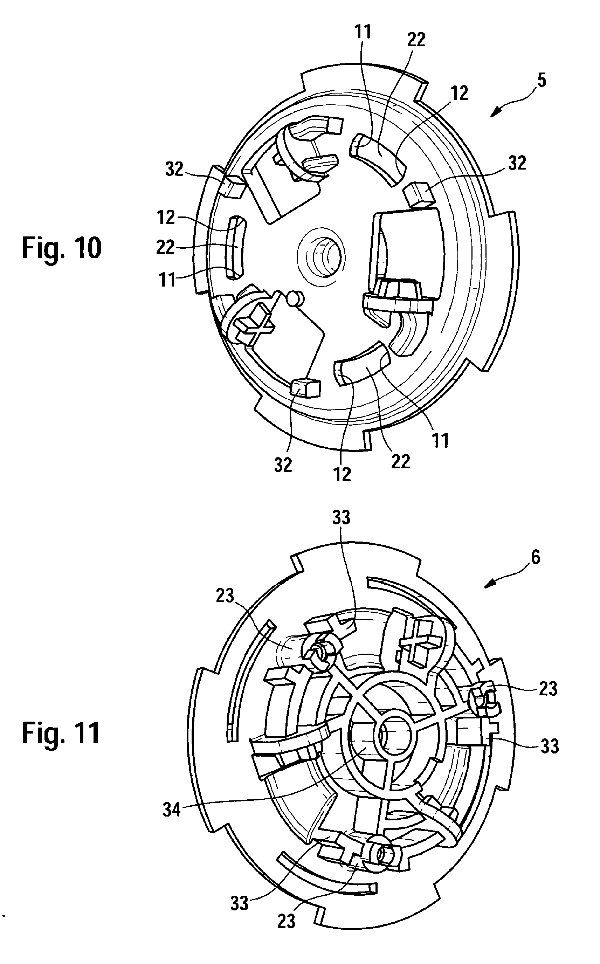

Die Figuren 8 bis 11 zeigen eine alternative Ausführungsform einer Kupplung 3. In diesem Falle werden als Federelemente Druckfedern 9 eingesetzt. Der Anschlag wird durch am ersten Kupplungselement 5 ausgebildete, dem zweiten Kupplungselement 6 zugewandte erste Stege 32 und durch am zweiten Kupplungselement 6 ausgebildete, dem ersten Kupplungselement 5 zugewandte zweite Stege 33 gebildet. In der Ausgangsstellung liegen die ersten Stege 32 durch die Vorspannung der Druckfedern 9 an den zweiten Stegen 33 an. Bei einer die Vorspannung der Druckfedern 9 übersteigenden Belastung der Kupplung 3 lösen sich im Zuge der gegenseitigen Verdrehung der Kupplungselemente 5, 6 die ersten Stege 32 von den zweiten Stegen 33, wie beispielsweise in Fig. 9 gezeigt. Bei zunehmender Verdrehung der Kupplungselemente 5, 6 schlagen ein oder mehrere Schrauben 31 gegen das Ende der jeweils zugeordneten Kulisse 22, wodurch eine Überbelastung der Kupplung 3 verhindert werden kann.

Das zweite Kupplungselement 6 weist eine mehrere Versteifungsrippen umfassende Versteifungsstruktur 34 auf, die das zweite Kupplungselement 6 versteift. Eine derartige Versteifungsstruktur eignet sich insbesondere für Kupplungselemente, die aus einem Kunststoff hergestellt sind. Auch Kupplungselemente aus Metall können grundsätzlich mit einer Versteifungsstruktur ausgebildet werden.

Im Übrigen entspricht die Kupplung 3 der im ersten Ausführungsbeispiel beschriebenen Kupplung 3.

In den Ausführungsbeispielen wurden die die Kupplungselemente verbindenden Federelemente durch Zugfedern 8 oder Druckfedern 9 ausgebildet. Alternativ können beispielsweise auch andere elastische Federelemente eingesetzt werden, beispielsweise Gasdruckfedern, Elastomerfedern, Biegefedern, Torsionsfedern oder Luftfedern. Das erfindungsgemäße Antriebssystem erfordert nicht zwingend die Verwendung eines bestimmten Typs von Federelement, grundsätzlich sind beliebige Federelemente einsetzbar. Des Weiteren kann die Zahl der verwendeten Federelemente variieren. Zu dem können ein oder mehrere Federelemente eingesetzt werden, die sowohl auf Zug als auch auf Druck belastbar sind. Damit ist es möglich, für beide Drehrichtungen der Kupplung 3 Belastungen, insbesondere das Überschreiten von maximalen Belastungen, zu registrieren.

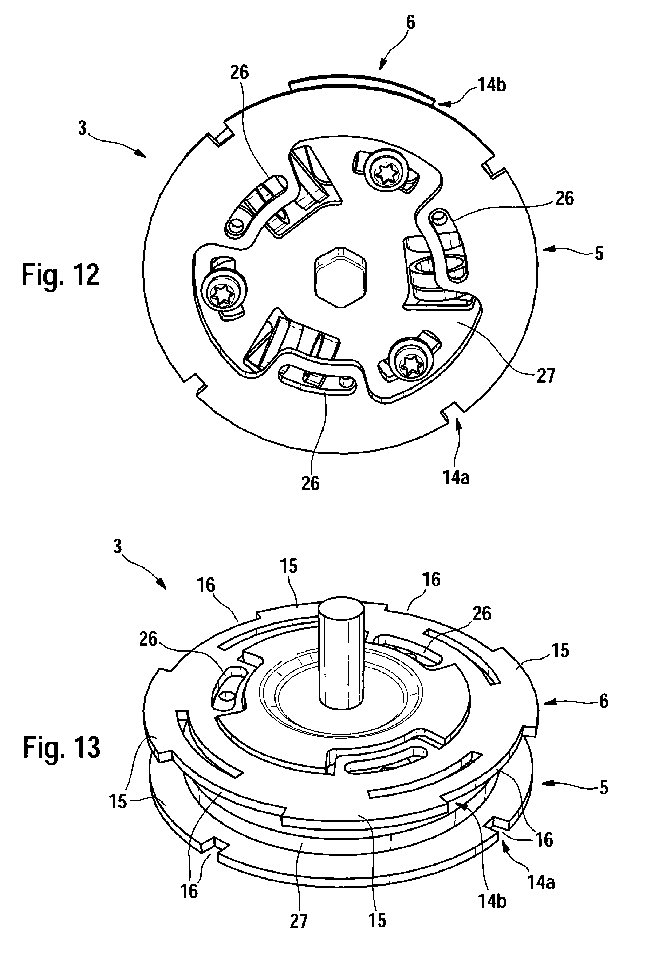

Die Figuren 12 bis 14 zeigen eine weitere alternative Ausführungsform der Kupplung 3. In diesem Falle sind die Spuren 14a und 14b gegeneinander verstellbar. Zum Verstellen der Spuren 14a, 14b sind mehrere Langlöcher 26 vorgesehen, die ein Verdrehen und ein Befestigen der Spuren 14a, 14b in einem durch die Langlöcher 26 vorgegebenen Winkelbereich gegenüber einem jeweiligen Trägerelement 27 des ersten Kupplungselementes 5 und des zweiten Kupplungselementes 6 ermöglichen. Die Befestigungsschrauben wurden der Übersicht wegen weggelassen. Die Verstellung der Spuren 14a, 14b ermöglicht es, die Phasendifferenz der Signale S1, S2, siehe Figuren 2, 3 zu verändern. Dies ermöglicht es beispielsweise, die Spuren 14a, 14b derart einzustellen, dass die Zeitdifferenz zwischen den beiden Signalen S1, S2 bei einer maximalen Belastung am kürzesten ist. Das Antriebssystem kann auf diese Weise beispielsweise für die Erkennung von maximalen Belastungen optimiert werden.

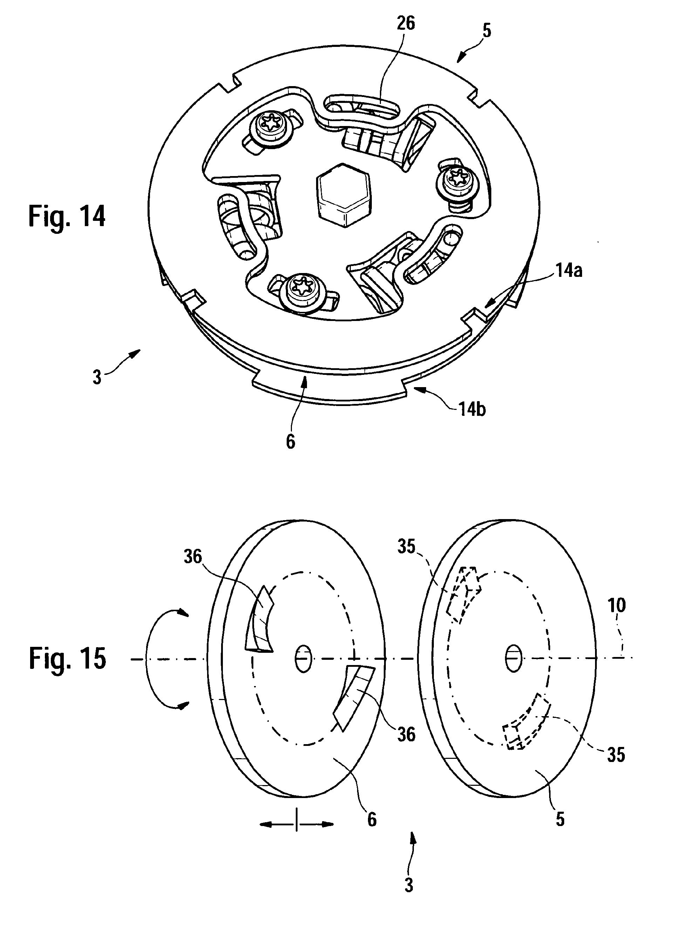

Die Fig. 15 zeigt eine weitere Ausführungsform einer Kupplung 3 in einer schematischen Explosionsdarstellung. Die Darstellung ist auf das erste Kupplungselement 5 und das zweite Kupplungselement 6 reduziert. Das erste Kupplungselement 5 umfasst zwei auf der dem zweiten Kupplungselement zugewandten Seite ausgebildete erste keilförmige Vorsprünge 35, das zweite Kupplungselement 6 umfasst zwei auf der dem ersten Kupplungselement 6 zugewandten Seite ausgebildete zweite keilförmige Vorsprünge 36. Im Zustand, in dem durch die Last 2 keine Belastung auf die Kupplung 3 ausgeübt wird, liegen die ersten keilförmigen Vorsprünge 35 im Wesentlichen deckend auf den zweiten keilförmigen Vorsprüngen 36. Bei einem Verdrehen des ersten Kupplungselementes 5 gegen das zweite Kupplungselement 6 gleiten die ersten und zweiten keilförmigen Vorsprünge übereinander ab, wodurch sich der Abstand zwischen den beiden Kupplungselementen in Abhängigkeit der Drehrichtung verringert oder erhöht. Die beiden Kupplungselemente 5, 6 sind mit einem oder mehreren hier nicht näher dargestellten Federelementen entlang der Drehachse 10 axial gegeneinander vorgespannt, so dass eine Erhöhung des Abstandes zwischen den Kupplungselementen 5, 6 gegen die Rückstellkraft der Federelemente erfolgt.

Der Abstand zwischen den beiden Kupplungselementen 5, 6 ist ein Maß für die Belastung, die eine Last 2 auf die Kupplung 3 ausübt. Sensoren für die Messung des Abstandes sind dem Fachmann hinlänglich bekannt. Die mit den Sensoren aufgenommenen Messwerte können wie in oben beschriebener Weise weiter verarbeitet werden.

Alternativ zu dem hier beschriebenen scheibenförmigen Aufbau der Kupplungselemente 5, 6 sind auch andere Formen für die Kupplungselemente 5, 6 denkbar. Beispielsweise können die Kupplungselemente 5, 6 durch zwei ineinander laufende Zylinder gebildet werden, die, wie in den vorhergehenden Ausführungsbeispielen beschrieben, miteinander durch ein oder mehrere Federelemente verbunden sind.

## Patentansprüche

1. Antriebssystem, umfassend einen Antrieb (1), eine Last (2), eine Kupplung (3) und eine Messvorrichtung (4), wobei die Kupplung (3) derart eingerichtet ist, dass diese eine von dem Antrieb (1) erzeugte Bewegung auf die Last (2) überträgt, wobei die Kupplung (3) ein antriebsseitiges erstes Kupplungselement (5) und ein lastseitiges zweites Kupplungselement (6) aufweist, und die Kupplung (3) derart eingerichtet ist, dass das erste Kupplungselement (5) und das zweite Kupplungselement (6) ihre Stellung in Abhängigkeit einer von der Last (2) auf die Kupplung (3) ausgeübten Belastung zueinander verändern, und die Messvorrichtung (4) derart eingerichtet ist, dass diese eine Änderung der Stellung des ersten Kupplungselementes (5) relativ zum zweiten Kupplungselement (6) misst.

2. Antriebssystem nach Anspruch 1, derart eingerichtet, dass das erste Kupplungselement (5) und das zweite Kupplungselement (6) bei einer von der Last (2) auf die Kupplung (3) ausgeübten Belastung von einer Ausgangsstellung in eine Belastungsstellung übergehen, in der die Stellung des ersten Kupplungselementes (5) relativ zum zweiten Kupplungselement (6) verändert ist, und bei einem Wegfall der Belastung das erste Kupplungselement (5) und das zweite Kupplungselement (6) in ihre Ausgangsstellung zurückkehren.

3. Antriebssystem nach Anspruch 1 oder 2, wobei das erste Kupplungselement (5) und das zweite Kupplungselement (6) durch mindestens ein Federelement (7, 8, 9) miteinander und gegeneinander beweglich verbunden sind, wobei das mindestens eine Federelement (7, 8, 9) bei einer von der Last (2) auf die Kupplung (3) ausgeübten Belastung elastisch deformiert wird.

4. Antriebssystem nach einem der vorhergehenden Ansprüche, wobei das erste Kupplungselement (5) und das zweite Kupplungselement (6) sich in Abhängigkeit einer von der Last (2) auf die Kupplung (3) ausgeübten Belastung gegeneinander verdrehen und/oder sich gegeneinander axial verschieben.

5. Antriebssystem nach einem der vorhergehenden Ansprüche, wobei das erste Kupplungselement (5) und das zweite Kupplungselement (6) auf einer gemeinsamen Drehachse (10) angeordnet sind.

6. Antriebssystem nach einem der vorhergehenden Ansprüche, wobei die Kupplung (3) mindestens einen Anschlag (11, 12) umfasst, der die relative Änderung der Stellung des ersten Kupplungselementes (5) gegenüber dem zweiten Kupplungselement (6) auf einen Maximalwert begrenzt.

7. Antriebssystem nach einem der vorhergehenden Ansprüche, wobei die Messvorrichtung (4) zur Messung einer Änderung der Stellung des ersten Kupplungselementes relativ zum zweiten Kupplungselement mindestens eine Lichtschranke (13a, 13b, 13c) umfasst.

8. Antriebssystem nach Anspruch 7, wobei die Messvorrichtung (4) zur Messung einer Änderung der Stellung des ersten Kupplungselementes (5) relativ zum zweiten Kupplungselement (6) mindestens erste Lichtschranke (13a) umfasst, die zur Messung der Stellung des ersten Kupplungselementes (5) angeordnet ist, und eine zweite Lichtschranke (13b, 13c) umfasst, die zur Messung der Stellung des zweiten Kupplungselementes (6) angeordnet ist.

9. Antriebssystem nach einem der Ansprüche 6 oder 7, wobei das erste Kupplungselement (5) zumindest eine erste Spur (14a) und das zweite Kupplungselement (6) zumindest eine zweite Spur (14b) aufweist, wobei die erste Spur (14a) und die zweite Spur (14b) geschlossene Bereiche (15) und offene Bereiche (16) aufweisen, die derart angeordnet sind, dass diese bei einer Drehbewegung des ersten Kupplungselementes (5) und/oder des zweiten Kupplungselementes (6) die mindestens eine Lichtschranke (13a, 13, 13c) abwechselnd unterbrechen und freigeben.

10. Antriebssystem nach einem der vorhergehenden Ansprüche, wobei das erste Kupplungselement (5) und/oder das zweite Kupplungselement (6) in Form einer Scheibe ausgebildet ist.

11. Antriebssystem nach einem der vorhergehenden Ansprüche, zusätzlich umfassend eine Steuervorrichtung (17), wobei die Steuereinrichtung (17) derart eingerichtet ist, dass diese überprüft, ob ein von der Messvorrichtung (4) gemessener Wert in einem vorgegebenen Wertebereich liegt, und im Falle dessen, dass der Wert außerhalb des Wertebereiches liegt, die vom Antrieb (1) erzeugte Bewegung reduziert, die vom Antrieb (1) erzeugte Bewegung stoppt und/oder ein Signal abgibt.

12. Antriebsystem nach einem der vorhergehenden Ansprüche, wobei der Antrieb (1) ein Elektromotor ist, vorzugsweise ein Schrittmotor ist oder ein Gleichstrommotor ist.

13. Antriebsystem nach einem der vorhergehenden Ansprüche, wobei die Last (2) eine medizinische Pumpe ist, beispielsweise eine medizinische Rollenpumpe oder eine medizinische Peristaltikpumpe ist.
